# EUROPEAN PATENT SPECIFICATION

(11) **EP 0 784 620 B1**
(45) Date of publication and mention of the grant of the patent: **08.12.1999**
(21) Application number: 95933400.4
(22) Date of filing: 19.09.1995
(51) Int. Cl.: C07D 405/12, C07D 405/14, C07D 211/46, A61K 31/445

(54) **N-SUBSTITUTED PIPERIDINYL BICYCLIC BENZOATE DERIVATIVES**
N-SUBSTITUIERTE PIPERIDINYL BICYCLISCHE BENZOATDERIVATE
DERIVES BENZOATES BICYCLIQUES DE PIPERIDINYLE N-SUBSTITUE

(30) Priority: 27.09.1994 EP 94202792
(43) Date of publication of application: 23.07.1997
(73) Proprietor: JANSSEN PHARMACEUTICA N.V., 2340 Beerse (BE)
(72) Inventor: VAN DAELE, Georges, Henri, Paul, DECEASED (BE); BOSMANS, Jean-Paul, René, Marie, André, B-2650 Edegem (BE); VAN DEN KEYBUS, Frans, Maria, Alfons, B-2190 Essen (BE)
(74) Representative: Wante, Dirk
(86) International application number: EP9503691
(87) International publication number: WO9610027

(56) References cited:
- EP-A- 0 309 043
- EP-A- 0 389 037
- EP-A- 0 445 862
- WO-A-93/03725
- WO-A-94/08995
- DE-A- 3 810 552
- KARL-HEINTZ BUCHHEIT ET AL.: "SDZ 205-557, a selective, surmountable antagonist for 5-HT4 receptors in the isolated guinea pig ileum", ARCHIVES OF PHARMACOLOGY, , , vol. 345, no. , pages 387 to 393-
- FRANK D. KING AND GARETH J. SANGER: "Gastrointestinal Mobility Enhancing Agents", ANNUAL REPORT IN MEDICINAL CHEMISTRY, , , vol. 23, no. , pages 201 to 210

## Description

The present invention is concerned with novel benzoate derivatives, pharmaceutical compositions comprising said novel compounds, processes for preparing compounds and compositions, and the use thereof as a medicine, in particular in the treatment of conditions involving a decreased motility of the colon.

In our EP-0,389,037-A, published on September 26, 1990, *N*-(3-hydroxy-4-piperidinyl) (dihydrobenzofuran or dihydro-2*H*-benzopyran)carboxamide derivatives are disclosed as having gastrointestinal motility stimulating properties. In our EP-0,445,862-A, published on September 11, 1991, *N*-(4-piperidinyl) (dihydrobenzofuran or dihydro-2*H*-benzopyran)carboxamide derivatives are disclosed also having gastrointestinal motility stimulating properties. WO 93/03725 (SmithKline Beecham), published on March 4, 1993, generically discloses the use as 5HT₄ receptor antagonists of esters of general formula X-CO-Y-Z, wherein X can be a substituted phenyl, Y can be oxygen, and Z can be a substituted piperidine moiety. WO 94/08995 (SmithKline Beecham), published on April 28, 1994 generically discloses, for instance, substituted 7-benzofuran carboxylates also having 5HT₄ antagonistic activity. The latter two patent applications describe the use of the 5HT₄ antagonistic compounds in the treatment of irritable bowel syndrome (IBS), in particular the diarrhoea aspects of IBS.

Unexpectedly, we have discovered that the present novel compounds show intestinal prokinetic activity. Hence, the presently disclosed compounds show utility in treatment of conditions involving a decreased motility of the intestine, especially the colon.

The present invention is concerned with novel benzoate derivatives having the formula 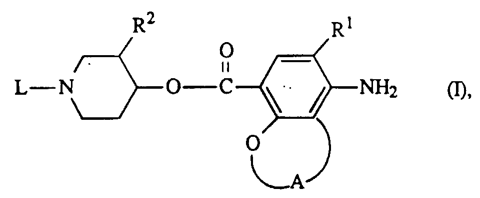 the *N*-oxide forms, the pharmaceutically acceptable acid addition salts and the stereochemically isomeric forms thereof, wherein :
R¹ is halo or C₁₋₆alkylsulfonylamino;
A represents a bivalent radical of formula :

   -CH₂-CH₂- (a),

   -CH₂-CH₂-CH₂- (b),

   -CH=CH- (c),

   in the radicals (a), (b) and (c) one or two hydrogen atoms may be replaced by a C₁₋₆alkyl;
R² is hydrogen or C₁₋₆alkyloxy;
L is a radical of formula :

   -Alk-R⁴ (d),

   -Alk-O-R⁵ (e),

   -Alk-NR⁶R⁷ (f);
Alk is C₁₋₁₂alkanediyl;
R⁴ is hydrogen; cyano; C₁₋₆alkylcarbonyl; C₁₋₆alkyloxycarbonyl; C₃₋₇cycloalkyl; C₁₋₆alkylsulfinyl; C₁₋₆alkylsulfonyl; phenyl or phenyl substituted with halo, C₁₋₆alkyl or C₁₋₆alkyloxy; tetrahydrofuran; dioxolane; dioxolane substituted with C₁₋₆alkyl; dioxane; dioxane substituted with C₁₋₆alkyl; pyridine; pyridine substituted with halo or C₁₋₆alkyl; pyridazine; pyridazine substituted with one or two substituents selected from halo, C₁₋₆alkyl, hydroxy; or a radical of formula : 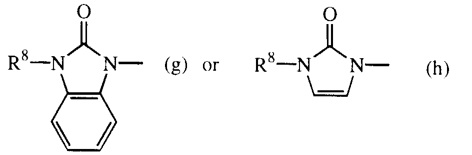 wherein R⁸ is hydrogen or C₁₋₆alkyl;
R⁵ is hydrogen; C₁₋₆alkyl; hydroxyC₁₋₆alkyl; C₁₋₆alkylcarbonyl; phenyl or phenyl substituted with up to three substituents selected from halo, C₁₋₆alkyl, C₁₋₆alkyloxy; R⁶ is hydrogen or C₁₋₆alkyl;
R⁷ is hydrogen; C₁₋₆alkyl; C₁₋₆alkylcarbonyl; C₁₋₆alkyloxycarbonyl; pyridazine; pyridazine substituted with one or two substituents selected from halo, C₁₋₆alkyl, hydroxy; pyrazine; pyrazine substituted with one or two substituents selected from halo, C₁₋₆alkyl, hydroxy; provided that when A is a bivalent radical of formula (c) and L is a radical of formula (d) then R⁴ is not hydrogen, cyano, C₁₋₆alkylcarbonyl, phenyl or phenyl substituted with halo, C₁₋₆alkyl or C₁₋₆alkyloxy; or when A is a bivalent radical of formula (c) and L is a radical of formula (e) then R⁵ is not hydrogen, C₁₋₆alkyl or phenyl; or when A is a bivalent radical of formula (c) and L is a radical of formula (f) then R⁷ is not C₁₋₆alkylcarbonyl.

As used in the foregoing definitions halo is generic to fluoro, chloro, bromo and iodo; C₁₋₄alkyl defines straight and branched chain saturated hydrocarbon radicals having from 1 to 4 carbon atoms such as, for example, methyl, ethyl, propyl, butyl, 1-methylethyl, 2-methylpropyl and the like; C₁₋₆alkyl is meant to include C₁₋₄alkyl and the higher homologues thereof having 5 or 6 carbon atoms, such as, for example, 2-methylbutyl, pentyl, hexyl and the like; C₃₋₇cycloalkyl is generic to cyclopropyl, cyclobutyl, cyclopentyl, cyclohexyl and cycloheptyl; C₁₋₁₂alkanediyl defines bivalent straight or branched chain hydrocarbon radicals containing from 1 to 12 carbon atoms such as, for example, 1,2-ethanediyl, 1,3-propanediyl, 1,4-butanediyl, 1,5-pentanediyl, 1,6-hexanediyl, 1,7-heptanediyl, 1,8-octanediyl, 1,9-nonanediyl, 1,10-decanediyl, 1,11-undecanediyl, 1,12-dodecanediyl and the branched isomers thereof.

The pharmaceutically acceptable acid addition salts as mentioned hereinabove are meant to comprise the therapeutically active non-toxic acid addition salt forms which the compounds of formula (I) are able to form. The latter can conveniently be obtained by treating the base form with such appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic, maleic, fumaric, malic, tartaric, citric, methane-sulfonic, ethanesulfonic, benzenesulfonic, *p* toluenesulfonic, cyclamic, salicylic, *p*-aminosalicylic, pamoic and the like acids. The term addition salt as used hereinabove also comprises the solvates which the compounds of formula (I) as well as the salts thereof, are able to form. Such solvates are for example hydrates, alcoholates and the like. Conversely the salt form can be converted by treatment with alkali into the free base form.

The term "stereochemically isomeric forms" as used hereinbefore defines all the possible isomeric forms which the compounds of formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the *cis-* or *trans*-configuration. Stereochemically isomeric forms of the compounds of formula (I) are obviously intended to be embraced within the scope of this invention.

Some of the compounds of formula (I) may also exist in their tautomeric form. Such forms although not explicitly indicated in the above formula are intended to be included within the scope of the present invention. For instance, compounds of formula (I) wherein R⁴ is 3- or 6-hydroxypyridazine, or a radical of formula (g) or (h) wherein R⁸ is hydrogen may exist in their corresponding tautomeric form.

The N-oxide forms of the compounds of formula (I) are meant to comprise those compounds of formula (I) wherein one or several nitrogen atoms are oxidized to the so-called *N*-oxide, particularly those *N*-oxides wherein the piperidine-nitrogen is *N*-oxidized
R¹ is interestingly halo, preferably chloro;
R² is interestingly hydrogen or C₁₋₄alkyloxy, preferably hydrogen or methoxy,
A is interestingly a bivalent radical of formula (a) or (b),
when A is substituted, methyl substitution is preferred;
when A is a bivalent radical of formula (a) or (b), geminal dimethyl substitution is preferred, especially on the carbon atom adjacent to the oxygen atom;
when L is a radical of formula (d), R⁴ is preferably hydrogen, cyano, C₁₋₆alkylcarbonyl, C₁₋₆alkyloxycarbonyl, C₃₋₇cycloalkyl, C₁₋₆alkylsulfonyl, tetrahydrofuran, dioxolane substituted with C₁₋₆alkyl, pyridine, a radical of formula (g) wherein R⁸ is C₁₋₆alkyl, pyridazine substituted with halo and hydroxy;
when L is a radical of formula (e), R⁵ is preferably hydrogen, C₁₋₆alkyl, hydroxyC₁₋₆alkyl, or phenyl substituted with halo;
when L is a radical of formula (f), R⁶ is preferably hydrogen and R⁷ is preferably hydrogen, or pyridazine substituted with C₁₋₆alkyl, C₁₋₆alkyloxycarbonyl.

Interesting compounds are those compounds of formula (I), wherein R¹ is chloro.

Further interesting compounds are those compounds of formula (I), wherein R² is hydrogen or methoxy.

More interesting compounds of formula (I) are those interesting compounds wherein A is a bivalent radical of formula (a) or (b).

Preferred compounds of formula (I) are :
1-[(tetrahydro-2-furanyl)methyl]-4-piperidinyl 4-amino-5-chloro-2,3-dihydro-7-benzofurancarboxylate;
1-[(tetrahydro-2-furanyl)methyl]-4-piperidinyl 5-amino-6-chloro-3,4-dihydro-2,2-dimethyl-2*H*-1-benzopyran-8-carboxylate;
1-(3-methoxypropyl)-4-piperidinyl 4-amino-5-chloro-2,3-dihydro-7-benzofuran-carboxylate;
1-[3-(2-methyl-1,3-dioxolan-2-yl)propyl]-4-piperidinyl 4-amino-5-chloro-2,3-dihydro-7-benzofurancarboxylate;
1-[3-(1-methylethoxy)propyl]-4-piperidinyl 4-amino-5-chloro-2,3-dihydro-7-benzo-furancarboxylate;
1-[2-(2-hydroxyethoxy)ethyl]-4-piperidinyl 4-amino-5-chloro-2,3-dihydro-7-benzo-furancarboxylate;
1-[3-(3-chloro-6-oxo-1(6*H*)-pyridazinyl)propyl]-4-piperidinyl 4-amino-5-chloro-2,3-dihydro-7-benzofurancarboxylate;
1-(4-oxopentyl)-4-piperidinyl 4-amino-5-chloro-2,3-dihydro-7-benzofurancarboxylate;
ethyl 4-[[(4-amino-5-chloro-2,3-dihydro-7-benzofuranyl)carbonyl]oxy]-1-piperidinebutanoate; and
1-[2-(tetrahydro-2-furanyl)ethyl]-4-piperidinyl 4-amino-5-chloro-2,3-dihydro-7-benzofurancarboxylate; the possible stereochemically isomeric forms thereof and the pharmaceutically acceptable acid addition salt thereof.

In order to simplify the structural representations of the compounds of formula (I) and certain starting materials and intermediates thereof, the radical 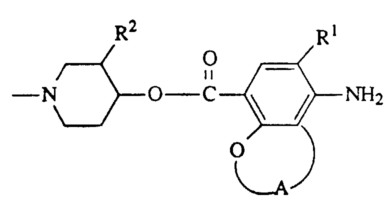 will hereafter be represented by the symbol D.

In the following preparations, the reaction products may be isolated from the reaction mixture and, if necessary, further purified according to methodologies generally known in the art such as, for example, extraction, distillation, crystallization, trituration and chromatography.

The compounds of formula (I) may be prepared by *N*-alkylating a piperidine of formula (II) with an intermediate of formula (III), wherein W¹ is an appropriate leaving group such as, for example, halo, e.g. chloro, bromo or iodo, or a sulfonyloxy group, e.g. methanesulfonyloxy, toluenesulfonyloxy and the like leaving groups. The *N*-alkylation reaction of (II) with (III) is conveniently conducted following art-known alkylation procedures.

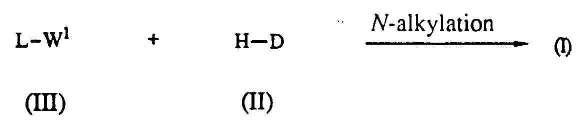

The compounds of formula (I) may also be prepared by the ester formation of an alcohol of formula (IV) with a carboxylic acid of formula (V) or a functional derivative thereof, such as an acylhalide, a symmetrical or mixed anhydride or an ester, preferably an activated ester, following art-known procedures.

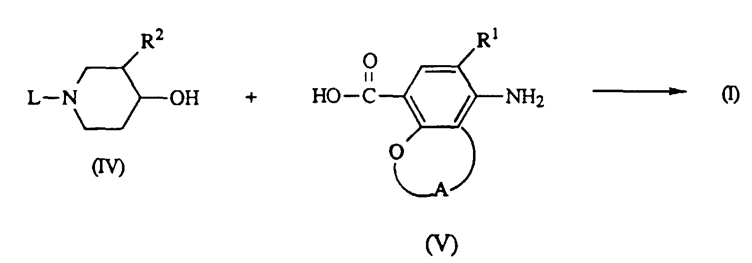

It may be expedient to protect amino or hydroxy groups, i.e. other than the reacting hydroxy groups, during the course of the reaction to avoid undesired side reactions. Said amino or hydroxy protecting group is removed after completion of the ester formation. Suitable protecting groups comprise readily removable groups such as C₁₋₄alkylcarbonyl, C₁₋₄alkyloxycarbonyl, phenylmethyl and the like protective groups.

The compounds of formula (I) may also be prepared by converting compounds of formula (I) into each other.

Compounds of formula (I), wherein L is a radical of formula (f), wherein R⁷ is other than hydrogen, said compounds being represented by formula (I-f-2), may be prepared by reacting a compound of formula (I), wherein R⁷ is hydrogen, said compounds being represented by formula (I-f-1) with a reagent of formula (VI), wherein W² is an appropriate leaving group such as, for example, halo, e.g. chloro, bromo or iodo, or a sulfonyloxy group, e.g. methanesulfonyloxy, toluenesulfonyloxy and the like leaving groups, following art-known reaction procedures.

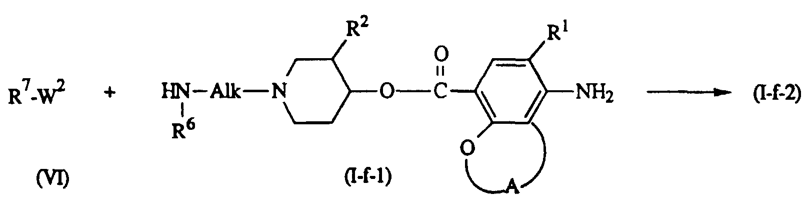

Compounds of formula (I), wherein L is a radical of formula (d), Alk is 1,2-ethanediyl and R⁴ is cyano may be prepared by reacting an intermediate of formula (II) with acrylonitrile, following art-known procedures.

Compounds of formula (I), wherein L is a radical of formula (e), Alk is 1,2-ethanediyl and R⁵ is hydrogen may be prepared by reacting an intermediate of formula (II) with oxirane following art-known reaction procedures.

The compounds of formula (I), wherein L is a radical of formula (f), wherein Alk is 1,3-propanediyl and wherein R⁶ and R⁷ is hydrogen may be prepared by hydrogenation of compounds of formula (I), wherein L is a radical of formula (d), Alk is 1,2-ethanediyl and R⁴ is cyano.

The compounds of formula (I) may also be converted to the corresponding *N*-oxide forms following art-known procedures for converting a trivalent nitrogen into its *N*-oxide form. Said *N*-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. t.butyl hydroperoxide. Suitable solvents are, for example, water, lower alkanols, e.g. ethanol and the like, hydrocarbons, e.g. methylbenzene, ketones, e.g. 2-butanone, halogenated hydrocarbons, e.g. dichloromethane, and mixtures of such solvents.

The intermediates of formula (II) may be derived from an appropriately substituted piperidine of formula (VII) with an intermediate acid of formula (V) or a functional derivative thereof, following art-known ester formation procedures, and subsequently removing the protective group P, following art-known procedures. P represents a readily removable protective group such as C₁₋₄alkylcarbonyl, C₁₋₄alkyloxycarbonyl, phenylmethyl and the like protective groups.

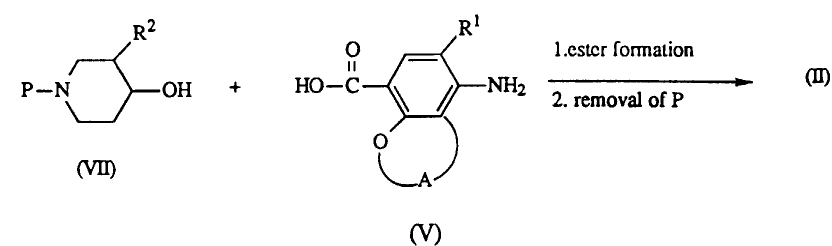

The preparation of intermediate acids of formula (V) is disclosed in EP-0,389,037-A.

The intermediates of formula (VII'), wherein P¹ represents P as well as hydrogen, may be prepared by reduction of an intermediate of formula (VIII) following art-known methods.

The intermediates of formula (VII'), wherein R² is C₁₋₆alkyloxy, said intermediates being represented by formula (VII'-a), and wherein R² and the 4-hydroxyl group have a *cis*-configuration may be prepared by reduction of an intermediate of formula (VIII-a) using a reductive agent such as substituted borohydrides, e.g. lithium tris*-sec*-butylborohydride, potassium tris-sec-butylborohydride, substituted aluminiumhydrides, lithium-tri-*tert*-butoxyaluminohydride and the like. Using stereochemically pure reagents said reduction may be performed in a stereospecific manner.

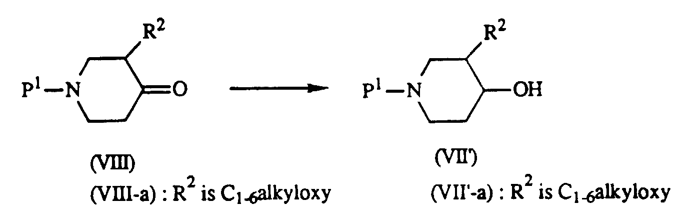

The *cis* and *trans* diastereomeric racemates of the compounds of formula (I), or any of the other intermediates may also be resolved into their optical isomers, *cis*(+)*, cis*(-), *trans*(+) and *trans*(-) by the application of art-known methodologies. Diastereoisomers may be separated by physical separation methods such as selective crystallization and chromatographic techniques, e.g. counter current distribution, and enantiomers may be separated from each other by the selective crystallization of their diastereomeric salts with enantiomerically pure acids or their enantiomerically pure derivatives.

The compounds of formula (I) and the intermediates of formula (II), the *N*-oxide forms, the pharmaceutically acceptable salts and stereoisomeric forms thereof possess favourable intestinal motility stimulating properties. In particular the present compounds show significant motility enhancing effects on the small and large intestine. The latter properties are evidenced by the results obtained in the "Guinea Pig Ileum Coaxial Stimulation" test and the "Colon motility in conscious dog" test. Both said tests are described hereinafter. Some of the compounds also show activity in the "Lidamidine test in dogs".

In view of their useful intestinal motility enhancing properties the subject compounds may be formulated into various forms for administration purposes.

To prepare the pharmaceutical compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) or (II) due to their increased water solubility over the corresponding base form, are obviously more suitable in the preparation of aqueous compositions. It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

In view of their capability to stimulate the motility of the intestinal system and in particular their capacity to enhance the motility of the colon, the subject compounds are useful to normalize or to improve the intestinal transit in subjects suffering from symptoms related to disturbed motility, e.g. a decreased peristalsis of the small and large intestine alone or in combination with delayed gastric emptying.
In view of the utility of the compounds of the present invention, there is provided a method of treating warm-blooded animals suffering from motility disorders of the intestinal system such as, for example, constipation, pseudo-obstruction, intestinal atony, post-operative intestinal atony, irritable bowel syndrome (IBS), drug-induced delayed transit, and in particular impaired colonic transit. Said method comprises the systemic administration of an effective intestinal stimulating amount of a compound of formula (I), a *N*-oxide, a pharmaceutically acceptable acid addition salt or a possible stereoisomeric form thereof, to warm-blooded animals. Hence, the use of a compound of formula (I) as medicine is provided, and in particular the use of a compound of formula (I) for the manufacture of a medicine for treating conditions involving a decreased motility of the colon.

In general it is contemplated that a therapeutically effective amount would be from about 0.001 mg/kg to about 10 mg/kg body weight, preferably from about 0.02 mg/kg to about 5 mg/kg body weight. A method of treatment may also include administering the active ingredient on a regimen of between two or four intakes per day.

The following examples are intended to illustrate and not to limit the scope of the present invention in all its aspects. Hereinafter "THF" means tetrahydrofuran and "DIPE" means diisopropylether.

### Experimental part

### A. Preparation of the intermediates

### Example 1

a) A solution of 3-methoxy-1-(phenylmethyl)-4-piperidinone (4.4 g) in THF was cooled to -75°C. Lithium tris-*sec*-butylborohydride was added dropwise action and the reaction mixture was stirred for 2 hours at -70°C. Acetic acid 10% (100 ml) was added dropwise at room temperature. The organic solvent was evaporated The aqueous residue was alkalized with NH₄OH, then extracted twice with DIPE. The separated organic layer was washed with water, dried over MgSO₄, filtered and the solvent was evaporated. The residue was purified by short column chromatography over silica gel (eluent : CH₂Cl₂/CH₃OH 95/5 upgrading to 98/2), yielding 1.3 g (29.4%) of *cis*-3-methoxy-1-(phenylmethyl)-4-piperidinol (intermediate 1)
b) A mixture of intermediate (1) (11.5 g) and methanol (150 ml) was hydrogenated at normal pressure and at room temperature with 2 g of palladium-on-charcoal catalyst 10%. After the calculated amount of hydrogen was taken up, the catalyst was filtered off and the filtrate was evaporated. The residue was purified by column-chromatography over silica gel (eluent : CHCl₃(CH₃OH/NH₃) 85/15). The pure fractions were collected and the eluent was evaporated, yielding 3.6 g (53%) of *cis*-3-methoxy-4-piperidinol as an oily residue (intermediate 2).
c) A solution of bis(1,1'-dimethylethyl)dicarbonate (65.5 g) in CHCl₃ (100 ml) was added dropwise to a solution of intermediate (2) (34 g) in CHCl₃ (350 ml) and the reaction mixture was stirred for 3 hours at room temperature. The reaction mixture was washed with water and ammonia, then with water. The separated organic layer was dried over MgSO₄, filtered and the solvent was evaporated. The residue (79 g) was purified by column chromatography over silica gel (eluent : CH₂Cl₂/(CH₃OH/NH₃) 97/3, upgrading to 95/5). The pure fractions were collected and the solvent was evaporated, yielding 58 g of (±)-1,1-dimethylethyl *cis*-4-hydroxy-3-methoxy-1-piperidinecarboxylate (96.4% crude residue) (intermediate 3).
d) Sodium hydride (6.2 g) was added to a solution of intermediate (3) (30 g) in THF (1000 ml). The mixture was stirred and refluxed under nitrogen flow for 3 hours, then cooled (solution I). 1,1'-carbonylbis-1*H*-imidazole (21 g) was added to a solution of 4-amino-5-chloro-2,3-dihydro-2,2-dimethyl-7-benzofurancarboxylic acid (31.4 g) in acetonitrile (1000 ml) and this mixture was stirred for 2 hours at room temperature. The solvent was evaporated. The residue was dissolved in THF (1000 ml), giving solution II. At room temperature, solution (II) was poured out into solution (I) and the reaction mixture was stirred for 2 hours at room temperature. The solvent was evaporated The residue was partitioned between CH₂Cl₂ and H₂O. The organic layer was separated and the aqueous layer was extracted twice with CH₂Cl₂. The separated organic layer was dried MgSO₄, filtered and the solvent was evaporated. The residue was purified by column chromatography over silica gel (eluent: CH₂Cl₂/CH₃OH 98/2). The desired fractions were collected and the solvent was evaporated, yielding 50 g of (±)-1,1-dimethylethyl *cis*-4-[[(4-amino-5-chloro-2,3-dihydro-2,2-dimethyl-7-benzofuranyl)-carbonyl]oxy]-3-methoxy-1-piperidinecarboxylate (85%) (intermediate 4).
e) A mixture of intermediate (4) (50 g) in THF (600 ml) and hydrochloric acid (60 ml) was stirred and refluxed for 30 minutes. The reaction mixture was cooled and alkalized with NH4OH. The separated aqueous layer was extracted with THF. The extract layer was evaporated and the residue was purified by column chromatography over silica gel (eluent: CH₂Cl₂/(CH₃OH/NH₃) 93/7). The pure fractions were collected and the solvent was evaporated. The residue was stirred in boiling DIPE. The mixture was cooled and the resulting precipitate was filtered off, dissolved in 2-propanol and converted into the ethanedioic acid salt (1:1) with ethanedioic acid (0.6 g). The mixture was boiled, cooled and the resulting precipitate was filtered off and dried, yielding 16 g of (±)-*cis*-3-methoxy-4-piperidinyl 4-amino-5-chloro-2,3-dihydro-2,2-dimethyl-7-benzofuran-carboxylate ethanedioate(1:1) (33%); mp. 193.2°C (intermediate 5).

In a similar manner were also prepared :
4-piperidinyl 4-amino-5-chloro-2,3-dihydro-2,2-dimethyl-7-benzofurancarboxylate;
mp. 161.0°C (intermediate 6).
(±)-*cis*-3-methoxy-4-piperidinyl 4-amino-5-chloro-2,3-dihydro-2,2-dimethyl-7-benzofurancarboxylate; (intermediate 7)
4-piperidinyl 4-amino-5-chloro-2,3-dihydro-7-benzofurancarboxylate; mp. 161.0°C (intermediate 8)

### B. Preparation of the final compounds

### Example 2

A mixture of 1-[3-(1-methylethoxy)propyl]-4-piperidinol (2.5 g) and *N,N*-dimethyl-4-pyridinamine (2 g) in dichloromethane (100 ml) was stirred at room temperature. 4-(acetylamino)-5-chloro-2,3-dihydro-7-benzofurancarbonyl chloride (2.7 g) was added and the reaction mixture was stirred for 72 hours at room temperature. The solvent was evaporated and the residue was purified by column chromatography over silica gel (eluent: CH₂Cl₂/CH₃OH 95/5). The desired fractions were collected and the solvent was evaporated. The residue (3.2 g) was dissolved in THF (100 ml) and treated with hydrochloric acid (10 ml). The reaction mixture was stirred and refluxed for 2 hours. The mixture was cooled and alkalized with NH₄OH. The organic solvent was evaporated and the aqueous residue was extracted twice with CH₂Cl₂. The separated organic layer was dried over MgSO₄, filtered and the solvent was evaporated. The residue was purified by short column chromatography over silica gel (eluent : CH₂Cl₂/ (CH₃OH/NH₃) 97/3). The desired fractions were collected and the solvent was evaporated. The residue (2.9 g) was purified by high-performance liquid chromatoraphy (eluent: CH₂Cl₂/(CH₃OH/NH₃)/CH₃OH 97/1/2). The pure fractions were collected and the solvent was evaporated. The residue was dissolved in 2-propanol and converted into the hydrochloric acid salt (1:1) with HCl/2-propanol. The mixture was boiled, then cooled. The precipitate was filtered off and dried (vacuum; 80 °C), yielding 0.50 g of 1-[3-(1-methylethoxy)propyl]-4-piperidinyl 4-amino-5-chloro-2,3-dihydro-7-benzofurancarboxylate monohydrochloride (12%); mp. 208.6°C (compound 4).

### Example 3

A mixture of intermediate (8) (3 g), 2-(3-chloropropyl)-2-methyl-1,3-dioxolane (2.5 g), sodium carbonate (2.1 g) and potassium iodide (catalytic quantity) in 4-methyl-2-pentanone (150 ml) was stirred and refluxed overnight. The mixture was cooled, washed with water, dried (MgSO₄), filtered and the solvent was evaporated. The residue was purified by column chromatography over silica gel (eluent : CH₂Cl₂, upgrading to CH₂Cl₂/(CH₃OH/NH₃) 97/3). The pure fractions were collected and the solvent was evaporated. The residue was stirred in boiling DIPE, cooled, stirred, filtered and recrystallized from CH₃CN/DIPE. The precipitate was filtered off and dried, yielding 1.00 g of 1-[3-(2-methyl-1,3-dioxolan-2-yl)propyl]-4-piperidinyl 4-amino-5-chloro-2,3-dihydro-7-benzofurancarboxylate (24%); mp. 128.1°C (compound 6).

### Example 4

A mixture of intermediate (5) (10 g) and 2-propenenitrile (2 ml) in 2-propanol (150 ml) was stirred and refluxed overnight. More 2-propenenitrile (1 ml) was added and the reaction mixture was stirred and refluxed for 20 hours. The solvent was evaporated The residue was purified by column chromatography over silica gel (eluent : CH₂Cl₂/(CH₃OH/NH₃) 97/3). The pure fractions were collected and the solvent was evaporated. The residue was stirred in boiling DIPE, cooled, stirred and the resulting precipitate was filtered off and dried (vacuum; 80 °C), yielding 10.7 g of (±)-*cis*-1-(2-cyanoethyl)-3-methoxy-4-piperidinyl 4-amino-5-chloro-2,3-dihydro-2,2-dimethyl-7-benzofurancarboxylate (94%); mp. 180.3°C (compound 27).

### Example 5

At room temperature, oxirane (gas) was allowed to bubble through a solution of intermediate (6) (3.3 g) in methanol (80 ml) for 3 hours keeping the temperature below 30 °C. The solvent was evaporated and the residue was purified by column chromatography over silica gel (eluent: CH₂Cl₂/(CH₃OH/NH₃) 93/7). The pure fractions were collected and the solvent was evaporated. The residue was stirred in boiling DIPE, cooled to room temperature and the precipitate was filtered off and dried (vacuum; 80 °C), yielding 1.66 g of 1-(2-hydroxyethyl)-4-piperidinyl 4-amino-5-chloro-2,3-dihydro-2,2-dimethyl-7-benzofurancarboxylate (45%); mp. 166.3°C (compound 21).

### Example 6

A mixture of compound (6) (2 g) in THF (50 ml) and hydrochloric acid (5 ml) was stirred and refluxed for 30 minutes. The reaction mixture was cooled and alkalized with NH₄OH. The separated aqueous layer was extracted with THF. The combined organic layers were evaporated and the residue was purified by column chromatography over silica gel (eluent: CH₂Cl₂/(CH₃OH/NH₃) 97/3). The pure fractions were collected and the solvent was evaporated. The residue was stirred in boiling DIPE. The precipitate was filtered off and dried and further purified by column chromatography over silica gel (eluent: CH₂Cl₂/CH₃OH 90/10). The pure fractions were collected and the solvent was evaporated, yielding 0.90 g of 1-(4-oxopentyl)-4-piperidinyl 4-amino-5-chloro-2,3-dihydro-7-benzofurancarboxylate (47%); mp. 104.8°C (compound 8).

### Example 7

A mixture of compound (9) (8.5 g) in THF (500 ml) was hydrogenated with Raney nickel catalyst (catalytic quantity) as a catalysL After uptake of H₂ (2 equiv), the catalyst was filtered off and the filtrate was evaporated. The residue was purified by column chromatography over silica gel (eluent: CH₂Cl₂/(CH₃OH/NH₃) 90/10). The pure fractions were collected and the solvent was evaporated. The residue was stirred in boiling DIPE, then cooled and the resulting precipitate was filtered off and dried, yielding 5.2 g of (±)-*cis*-1-(2-aminoethyl)-3-methoxy-4-piperidinyl 4-amino-5-chloro-2,3-dihydro-7-benzofurancarboxylate (61%); mp. 133.9°C (compound 11).

### Example 8

A mixture of compound (11) (4 g), 2-chloro-3-methylpyrazine (2.8 g) and *N,N*-diethylethanamine (2.8 ml) was stirred for 24 hours at 120 °C. The mixture was cooled and dissolved in CH₂Cl₂. The organic solution was purified twice by column chromatography over silica gel (eluent: CH₂Cl₂/(CH₃OH/NH₃) 95/5). The pure fractions were collected and the solvent was evaporated. The residue was stirred in boiling DIPE, cooled and the resulting precipitate was filtered off and dried, yielding 0.53 g of (±)-*cis*-3-methoxy-1-[2-[(3-methyl-2-pyrazinyl)amino]ethyl]-4-piperidinyl 4-amino-5-chloro-2,3-dihydro-7-benzofurancarboxylate (11.5%); mp. 124.1°C (compound 12).

### Example 9

A mixture of compound (38) (4.5 g), 2-chloro-3-methylpyrazine (3.3 g) and *N,N*-diethylethanamine (2.1 ml) was stirred for 20 hours at 120 °C. The reaction mixture was cooled and purified by column chromatography over silica gel (eluent : CH₂Cl₂/(CH₃OH/NH₃) 95/5). The desired fractions were collected and the solvent was evaporated. The residue was repurified by column chromatography over silica gel (eluent: CH₂Cl₂/CH₃OH 90/10). The pure fractions were collected and the solvent was evaporated. The residue was solidified in DIPE. The precipitate was filtered off and dried, yielding 2.10 g of 1-[2-[(3-methyl-2-pyrazinyl)amino]ethyl]-4-piperidinyl 4-amino-5-chloro-2,3-dihydro-7-benzofurancarboxylate (38%); mp. 108.6°C (compound 39).

Tables 1 through 3 list compounds that were prepared in a similar way as in one of the hereinabove mentioned examples.

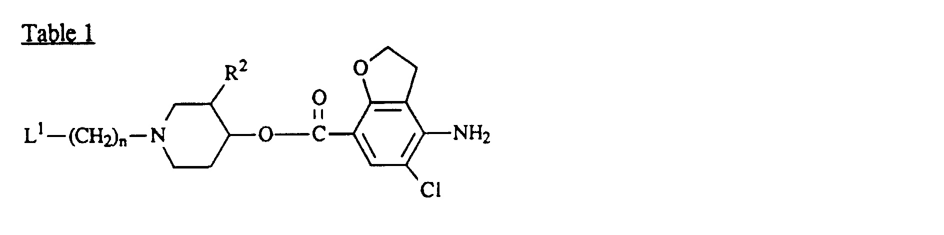

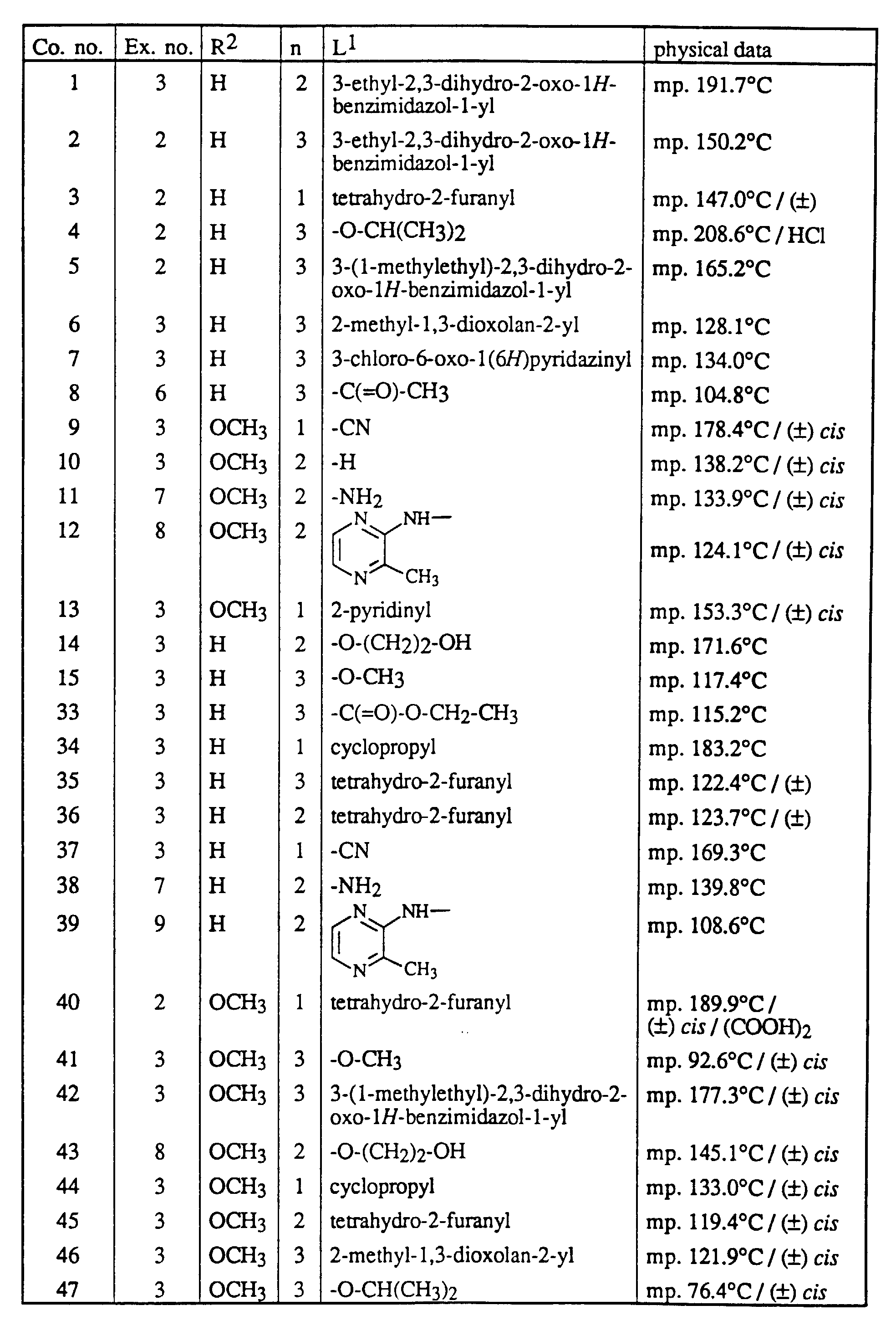

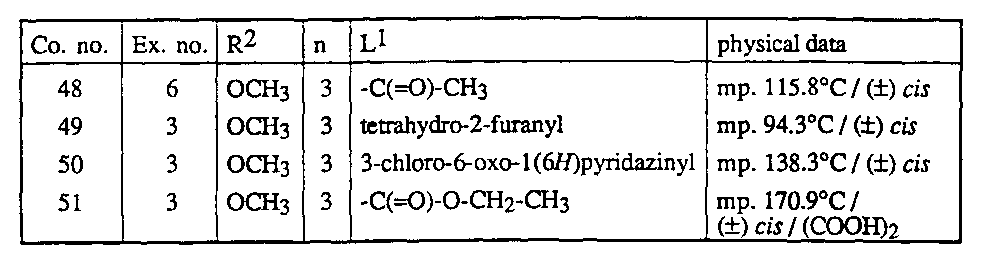

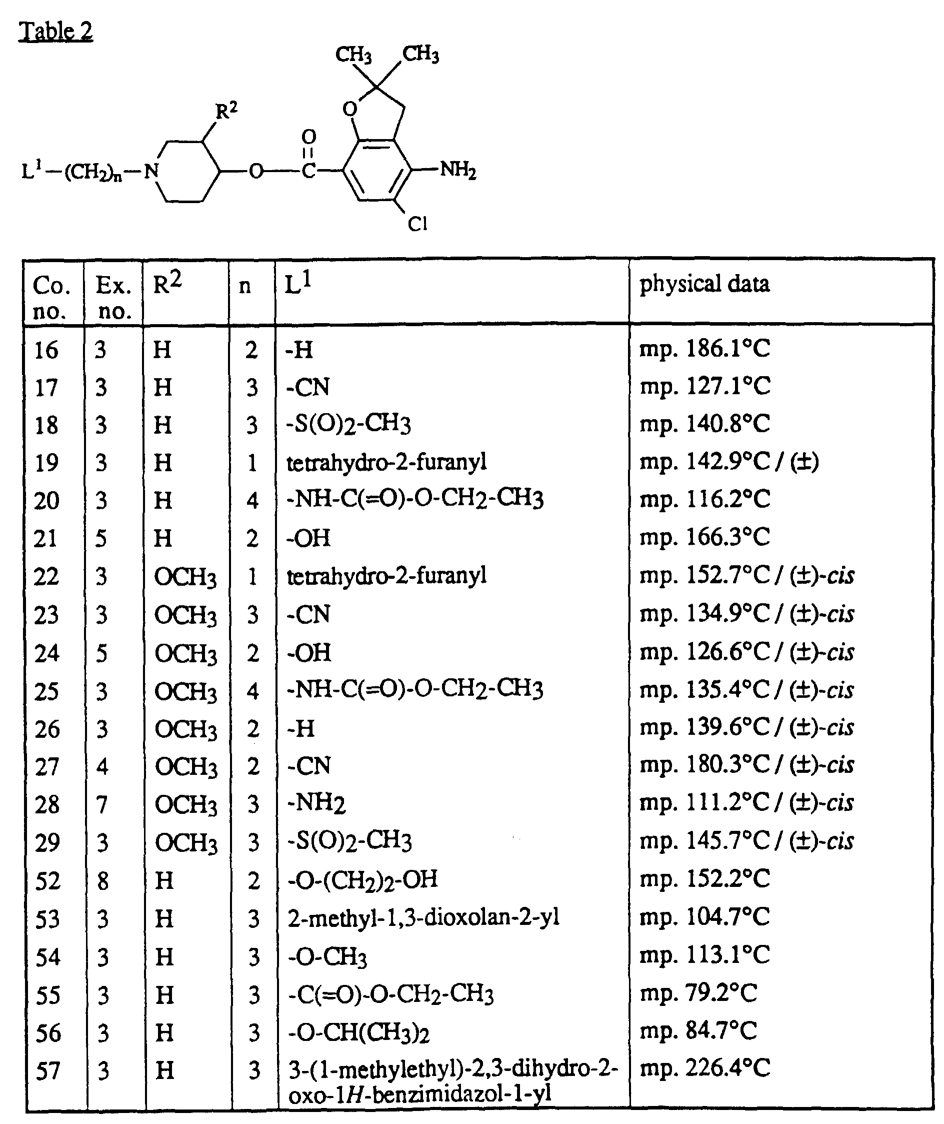

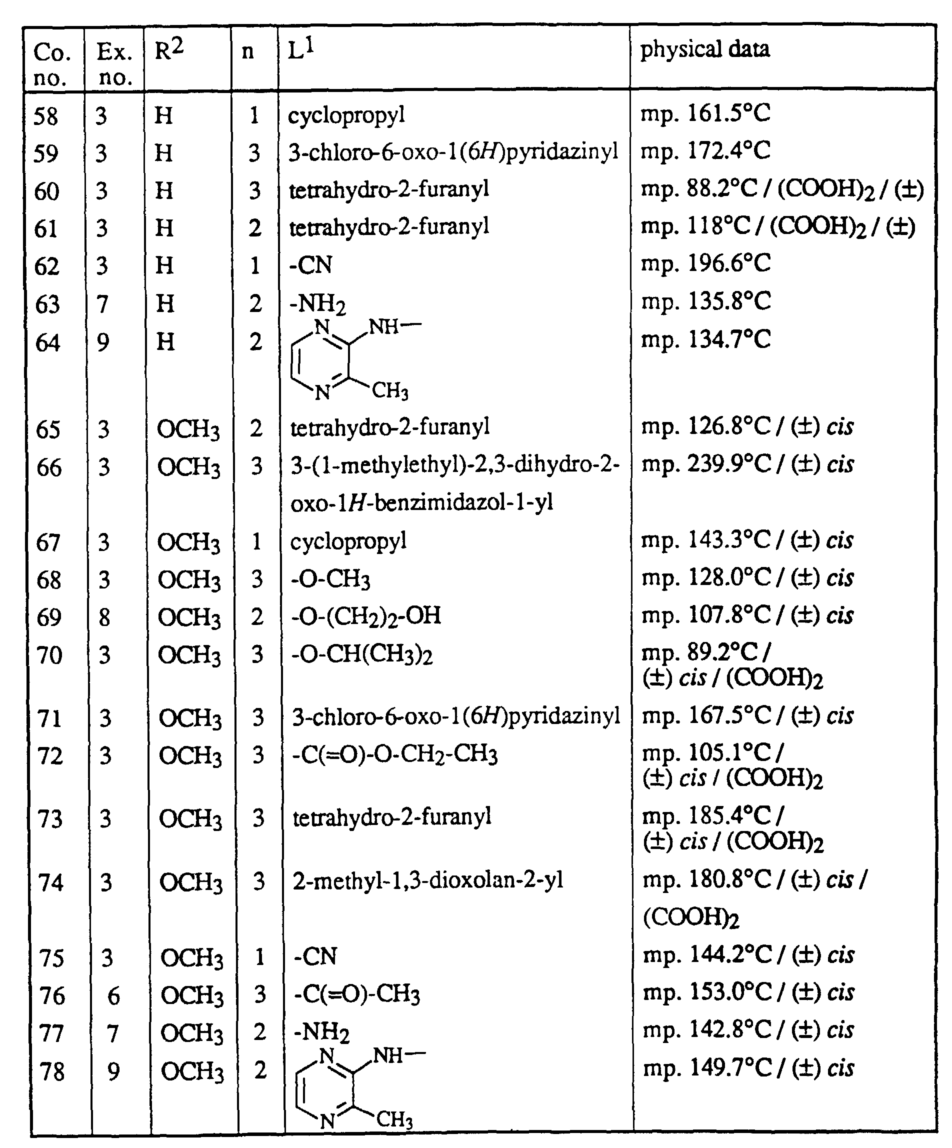

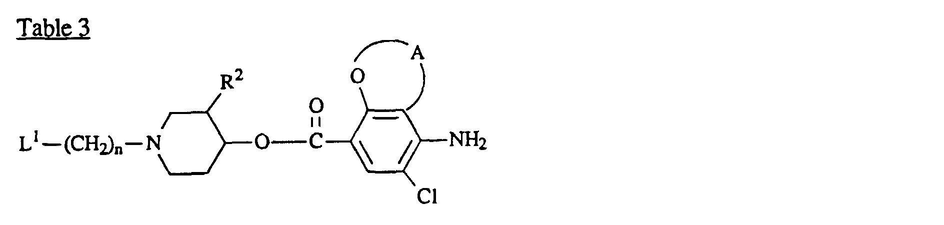

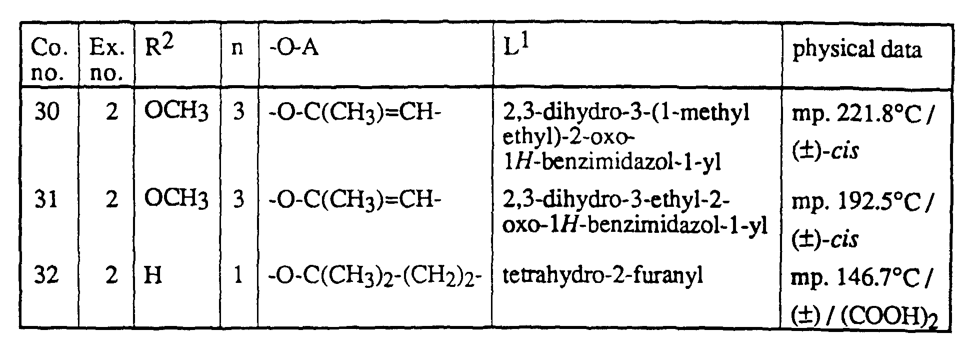

### C. Pharmacological examples

### Example 10 : Guinea Pig Ileum Coaxial Stimulation.

Dunkin Hartley guinea-pigs of both sexes (body weight ± 500 g) were killed by decapitation. The ileum was removed and cleansed with warmed and oxygenated Krebs-Henseleit solution. Non-terminal, intact ileum segments, 4.5 cm long, of the guinea pig were vertically suspended with a preload of 1 g in 100 ml Krebs-Henseleit solution (37.5°C), gassed with a mixture of 95% O₂ and 5% CO₂. Transmural excitation was applied over the whole length of the ileum segment by means of two platinum electrodes, the anode threaded through the lumen of the ileum, the cathode in the bathing solution. The preparation was excited with single rectangular stimili [1 msec; 0.1 Hz; submaximal response (current leading to 80% of maximal reponse)] from a progammable stimulator. Contractions were measured isometrically. During the stabilization period of 30 min, the strips were repeatedly stretched to a tension of 2g, in order to obtain a steady state tension of lg. Before starting the electrical stimulation, a cumulative dose response curve of acetylcholine was given. The electrical stimulation was started at supramaximal current to determine the maximal amplitude of the twitch responses. When these responses were stable, a submaximal stimulation to obtain 80% of the maximal responses was given until the twitch responses were constant for at least 15 min, whereafter a single dose of the test compound was added to the bath fluid. The amplitude of the twitch response five minutes after the administration of the test compound is compared with the amplitude before the administration of the test compound. The compounds with number 1, 4, 6-8, 14-16, 32-36, 39, 46 and 50 showed an increase of the amplitude of the twitch response of more than 5 % at a concentration of 3.10⁻⁹ M.

### Example 11: Motility of the colon in the conscious dog.

Female beagle dogs, weighing 7-17 kg, were implanted with isometric force transducers, under general anaesthesia and aseptic precautions. To study the colonic motility, transducers were sutured on the colon at 8, 16, 24 and 32 cm distance from the ileo-caecal-valve. Dogs were allowed a recovery period of at least two weeks. Experiments were started after a fasting period of ± 20 hours, during which water was available *ad libitum*. During the experiments, dogs were free to move in their cages, thanks to the telemetric (wireless) system. The cages were built in a special room, provided with glass pervious to light in one direction, i.e. the observator can see the dogs while the dogs can not see the observator. Via this system it was possible to observe the dogs for behavioral changes and to determine defecation events. The information from the transduceres was transmitted in digitized form by a small, specially built transmitter box. This box was placed in a jacket worn by the dog. The signals were received via a microphone above each cage and were transmitted to a central computer system.
One of the parameters in this test is the defecation of the dogs. During the first three hours after administration of the test compound, the dogs were observed to determine whether and when defecation occurred. Compounds of the present invention induced defecation in the test animals during those first three hours.

### D. Composition examples

The following formulations exemplify typical pharmaceutical compositions in dosage unit form suitable for systemic or topical administration to warm-blooded animals in accordance with the present invention.
"Active ingredient" (A.I.) as used throughout these examples relates to a compound of formula (I), a *N*-oxide form, a pharmaceutically acceptable acid addition salt or a stereochemically isomeric form thereof.

### Example 12 : Oral solutions

9 g of methyl 4-hydroxybenzoate and 1 g of propyl 4-hydroxybenzoate are dissolved in 4 l of boiling purified water. In 3 l of this solution are dissolved first 10 g of 2,3-dihydroxybutanedioic acid and thereafter 20 g of the A.I. The latter solution is combined with the remaining part of the former solution and 12 l of 1,2,3-propanetriol and 3 l of sorbitol 70% solution are added thereto. 40 g of sodium saccharin are dissolved in 0.5 l of water and 2 ml of raspberry and 2 ml of gooseberry essence are added. The latter solution is combined with the former, water is added q.s. to a volume of 20 l providing an oral solution comprising 5 mg of the A.I. per teaspoonful (5 ml). The resulting solution is filled in suitable containers.

### Example 13 : Capsules

20 g of the A.I., 6 g sodium lauryl sulfate, 56 g starch, 56 g lactose, 0.8 g colloidal silicon dioxide, and 1.2 g magnesium stearate are vigorously stirred together. The resulting mixture is subsequently filled into 1000 suitable hardened gelatin capsules, each comprising 20 mg of the A.I..

### Example 14 : Film-coated tablets

### Preparation of tablet core

A mixture of 100 g of the A.I., 570 g lactose and 200 g starch is mixed well and thereafter humidified with a solution of 5 g sodium dodecyl sulfate and 10 g polyvinylpyrrolidone in about 200 ml of water. The wet powder mixture is sieved, dried and sieved again. Then there are added 100 g microcrystalline cellulose and 15 g hydrogenated vegetable oil. The whole is mixed well and compressed into tablets, giving 10.000 tablets, each comprising 10 mg of the active ingredient.

### Coating

To a solution of 10 g methyl cellulose in 75 ml of denaturated ethanol there is added a solution of 5 g of ethyl cellulose in 150 ml of dichloromethane. Then there are added 75 ml of dichloromethane and 2.5 ml 1,2,3-propanetriol. 10 g of polyethylene glycol is molten and dissolved in 75 ml of dichloromethane. The latter solution is added to the former and then there are added 2.5 g of magnesium octadecanoate, 5 g of polyvinylpyrrolidone and 30 ml of concentrated colour suspension and the whole is homogenated. The tablet cores are coated with the thus obtained mixture in a coating apparatus.

### Example 15 : Injectable solution

1.8 g methyl 4-hydroxybenzoate and 0.2 g propyl 4-hydroxybenzoate were dissolved in about 0.5 1 of boiling water for injection. After cooling to about 50°C there were added while stirring 4 g lactic acid, 0.05 g propylene glycol and 4 g of the A.I. The solution was cooled to room temperature and supplemented with water for injection q.s. ad 1 l volume, giving a solution of 4 mg/ml of A.I. The solution was sterilized by filtration (U.S.P. XVII p. 811) and filled in sterile containers.

### Example 16: Suppositories

3 g A.I. was dissolved in a solution of 3 g 2,3-dihydroxy-butanedioic acid in 25 ml polyethylene glycol 400. 12 G surfactant and triglycerides q.s. ad 300 g were molten together. The latter mixture was mixed well with the former solution. The thus obtained mixture was poured into moulds at a temperature of 37∼38°C to form 100 suppositories each containing 30 mg of the active ingredient.

## Claims

1. A compound having the formula 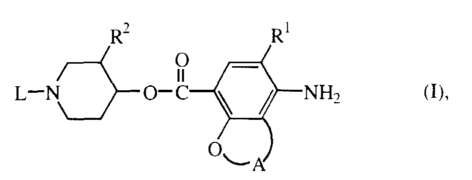 a *N*-oxide form, a pharmaceutically acceptable acid addition salt or a stereochemically isomeric form thereof, wherein
R¹ is halo or C₁₋₆alkylsulfonylamino;
A represents a bivalent radical of formula :
-CH₂-CH₂- (a),
-CH₂-CH₂-CH₂- (b),
-CH=CH- (c),
in the radicals (a), (b) and (c) one or two hydrogen atoms may be replaced by a C₁₋₆alkyl;
R² is hydrogen or C₁₋₆alkyloxy;
L is a radical of formula :
-Alk-R⁴ (d),
-Alk-O-R⁵ (e),
-Alk-NR⁶R⁷ (f),
Alk is C₁₋₁₂alkanediyl;
R⁴ is hydrogen; cyano; C₁₋₆alkylcarbonyl; C₁₋₆alkyloxycarbonyl; C₃₋₇cycloalkyl; C₁₋₆alkylsulfinyl; C₁₋₆alkylsulfonyl; phenyl or phenyl substituted with halo, C₁₋₆alkyl or C₁₋₆alkyloxy; tetrahydrofuran; dioxolane; dioxolane substituted with C₁₋₆alkyl; dioxane; dioxane substituted with C₁₋₆alkyl; pyridine; pyridine substituted with halo or C₁₋₆alkyl; pyridazine; pyridazine substituted with one or two substituents selected from halo, C₁₋₆alkyl, hydroxy; or a radical of formula : 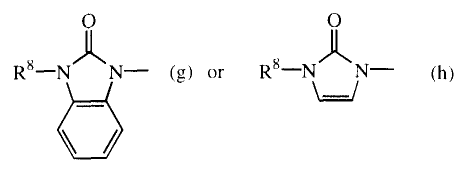 wherein R⁸ is hydrogen or C₁₋₆alkyl;
R⁵ is hydrogen; C₁₋₆alkyl; hydroxyC₁₋₆alkyl; C₁₋₆alkylcarbonyl; phenyl or phenyl substituted with up to three substituents selected from halo, C₁₋₆alkyl, C₁₋₆alkyloxy;
R⁶ is hydrogen or C₁₋₆alkyl;
R⁷ is hydrogen; C₁₋₆alkyl; C₁₋₆alkylcarbonyl; C₁₋₆alkyloxycarbonyl; pyridazine; pyridazine substituted with one or two substituents selected from halo, C₁₋₆alkyl, hydroxy; pyrazine; pyrazine substituted with one or two substituents selected from halo, C₁₋₆alkyl, hydroxy;
provided that when A is a bivalent radical of formula (c) and L is a radical of formula (d) then R⁴ is not hydrogen, cyano, C₁₋₆alkylcarbonyl, phenyl or phenyl substituted with halo, C₁₋₆alkyl or C₁₋₆alkyloxy; or when A is a bivalent radical of formula (c) and L is a radical of formula (e) then R⁵ is not hydrogen, C₁₋₆alkyl or phenyl; or when A is a bivalent radical of formula (c) and L is a radical of formula (f) then R⁷ is not C₁₋₆alkylcarbonyl.

2. A compound as claimed in claim 1 wherein R⁴ is hydrogen; cyano; C₁₋₆alkylcarbonyl; C₁₋₆alkylsulfinyl; C₁₋₆alkylsulfonyl; phenyl or phenyl substituted with halo, C₁₋₆alkyl or C₁₋₆alkyloxy; tetrahydrofuran; dioxolane; dioxolane substituted with C₁₋₆alkyl; dioxane; dioxane substituted with C₁₋₆alkyl; pyridine; pyridine substituted with halo or C₁₋₆alkyl; pyridazine; pyridazine substituted with one or two substituents selected from halo, C₁₋₆alkyl, hydroxy; or a radical of formula : 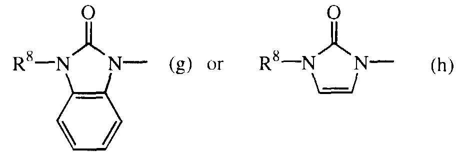 wherein R⁸ is hydrogen or C₁₋₆alkyl.

3. A compound as claimed in claim 1 wherein R¹ is chloro.

4. A compound as claimed in claim 1 wherein R² is hydrogen or methoxy.

5. A compound as claimed in claim 1 wherein the compound is selected from 1-[(tetrahydro-2-furanyl)methyl]-4-piperidinyl 4-amino-5-chloro-2,3-dihydro-7-benzofurancarboxylate; 1-[(tetrahydro-2-furanyl)methyl]-4-piperidinyl 5-amino-6-chloro-3,4-dihydro-2,2-dimethyl-2*H*-1-benzopyran-8-carboxylate; 1-(3-methoxypropyl)-4-piperidinyl 4-amino-5-chloro-2,3-dihydro-7-benzofurancarboxylate; 1-[3-(2-methyl-1,3-dioxolan-2-yl)propyl]-4-piperidinyl 4-amino-5-chloro-2,3-dihydro-7-benzofurancarboxylate; 1-[3-(1-methylethoxy )propyl]-4-piperidinyl 4-amino-5-chloro-2,3-dihydro-7-benzofurancarboxylate; 1-[2-(2-hydroxyethoxy)-ethyl]-4-piperidinyl 4-amino-5-chloro-2,3-dihydro-7-benzofurancarboxylate; 1-[3-(3-chloro-6-oxo-1(6*H*)-pyridazinyl)propyl]-4-piperidinyl 4-amino-5-chloro-2,3-dihydro-7-benzofurancarboxylate; 1-(4-oxopentyl)-4-piperidinyl 4-amino-5-chloro-2,3-dihydro-7-benzofurancarboxylate; ethyl 4-[[(4-amino-5-chloro-2,3-dihydro-7-benzofuranyl)carbonyl]oxy]-1-piperidinebutanoate; and 1-[2-(tetrahydro-2-furanyl)ethyl]-4-piperidinyl 4-amino-5-chloro-2,3-dihydro-7-benzofurancarboxylate; a stereochemically isomeric form thereof or a pharmaceutically acceptable acid addition salt thereof.

6. A pharmaceutical composition comprising a therapeuticaly effective amount of a compound as claimed in claim 1 and a pharmaceutically acceptable carrier.

7. A process for preparing a pharmaceutical composition as claimed in claim 6 wherein the compound as claimed in claim 1 is intimately mixed with a pharmaceutically acceptable carrier.

8. A compound as claimed in claim 1 for use as a medicine.

9. Use of a compound as claimed in claim 1 for the manufacture of a medicament for the treatment of intestinal disorders involving a decreased motility of the colon.

10. A process for preparing a compound as claimed in claim 1 wherein
a) a piperidine of formula (II) is *N*-alkylated with an intermediate of formula (III), 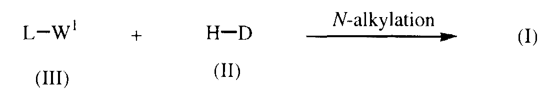 wherein L is as defined in claim 1, W¹ is an appropriate leaving group and D is a radical of formula 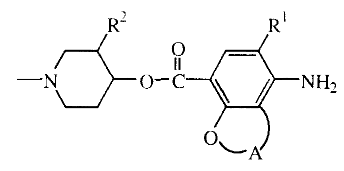 wherein R¹, R² and A are defined as in claim 1;
b) an alcohol of formula (IV) is reacted with a carboxylic acid of formula (V) or a functional derivative thereof, such as an acylhalide, a symmetrical or mixed anhydride or an ester; 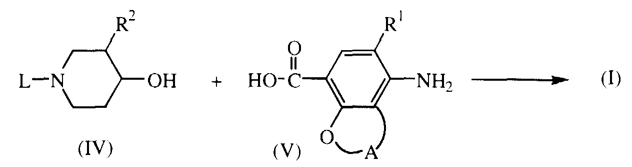 wherein R¹, R², L and A are defined as in claim 1;
and optionally converting the compounds of formula (I) into each other by a functional group transformation reaction; and, if desired, converting a compound of formula (I) into a therapeutically active non-toxic acid addition salt, or conversely, converting an acid addition salt into a free base form with alkali; and/ or preparing stereochemically isomeric forms thereof.

## Patentansprüche

1. Verbindung mit der Formel 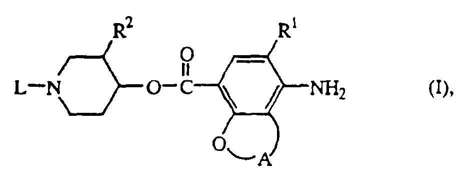 eine *N*-Oxidform, ein pharmazeutisch unbedenkliches Säureadditionssalz oder eine stereochemisch isomere Form davon, wobei
R¹ für Halogen oder C₁₋₆-Alkylsulfonylamino;
A für einen zweiwertigen Rest der Formel
-CH₂-CH₂- (a),
-CH₂-CH₂-CH₂- (b)
oder
-CH=CH- (c),
wobei in den Resten (a), (b) und (c) ein oder zwei Wasserstoffatome durch C₁₋₆-Alkyl ersetzt sein können;
R² für Wasserstoff oder C₁₋₆-Alkyloxy;
L für einen Rest der Formel:
-Alk-R⁴ (d),
-Alk-O-R⁵ (e)
oder
-Alk-NR⁶R⁷ (f),
worin Alk C₁₋₁₂-Alkandiyl bedeutet;
R⁴ für Wasserstoff; Cyano; C₁₋₆-Alkylcarbonyl; C₁₋₆-Alkyloxycarbonyl; C₃₋₇-Cycloalkyl; C₁₋₆-Alkylsulfinyl; C₁₋₆-Alkylsulfonyl; gegebenenfalls mit Halogen, C₁₋₆-Alkyl oder C₁₋₆-Alkyloxy substituiertes Phenyl; Tetrahydrofuran; Dioxolan; mit C₁₋₆-Alkyl substituiertes Dioxolan; Dioxan; mit C₁₋₆-Alkyl substituiertes Dioxan; Pyridin; mit Halogen oder C₁₋₆-Alkyl substituiertes Pyridin; Pyridazin; mit einem oder zwei unter Halogen, C₁₋₆-Alkyl und Hydroxy ausgewählten Substituenten substituiertes Pyridazin oder einen Rest der Formel: 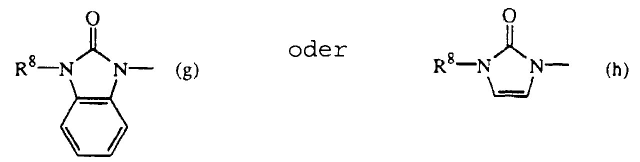 worin R⁸ Wasserstoff oder C₁₋₆-Alkyl bedeutet;
R⁵ für Wasserstoff; C₁₋₆-Alkyl; Hydroxy-C₁₋₆-alkyl; C₁₋₆-Alkylcarbonyl oder gegebenenfalls mit bis zu drei unter Halogen, C₁₋₆-Alkyl und C₁₋₆-Alkyloxy ausgewählten Substituenten substituiertes Phenyl;
R⁶ für Wasserstoff oder C₁₋₆-Alkyl und
R⁷ für Wasserstoff; C₁₋₆-Alkyl; C₁₋₆-Alkylcarbonyl; C₁₋₆-Alkyloxycarbonyl; Pyridazin; mit einem oder zwei unter Halogen, C₁₋₆-Alkyl und Hydroxy ausgewählten Substituenten substituiertes Pyridazin; Pyrazin oder mit einem oder zwei unter Halogen, C₁₋₆-Alkyl und Hydroxy ausgewählten Substituenten substituiertes Pyrazin steht; mit der Maßgabe, daß für den Fall, daß A für einen zweiwertigen Rest der Formel (c) und L für einen Rest der Formel (d) steht, R⁴ nicht für Wasserstoff, Cyano, C₁₋₆-Alkylcarbonyl oder gegebenenfalls mit Halogen, C₁₋₆-Alkyl oder C₁₋₆-Alkyloxy substituiertes Phenyl steht; bzw. für den Fall, daß A für einen zweiwertigen Rest der Formel (c) und L für einen Rest der Formel (e) steht, R⁵ nicht für Wasserstoff, C₁₋₆-Alkyl oder Phenyl steht; bzw. für den Fall, daß A für einen zweiwertigen Rest der Formel (c) und L für einen Rest der Formel (f) steht, R⁷ nicht für C₁₋₆-Alkylcarbonyl steht.

2. Verbindung nach Anspruch 1, in der R⁴ für Wasserstoff; Cyano; C₁₋₆-Alkylcarbonyl; C₁₋₆-Alkylsulfinyl; C₁₋₆-Alkylsulfonyl; gegebenenfalls mit Halogen, C₁₋₆-Alkyl oder C₁₋₆-Alkyloxy substituiertes Phenyl; Tetrahydrofuran; Dioxolan; mit C₁₋₆-Alkyl substituiertes Dioxolan; Dioxan; mit C₁₋₆-Alkyl substituiertes Dioxan; Pyridin; mit Halogen oder C₁₋₆-Alkyl substituiertes Pyridin; Pyridazin; mit einem oder zwei unter Halogen, C₁₋₆-Alkyl und Hydroxy ausgewählten Substituenten substituiertes Pyridazin oder einen Rest der Formel: 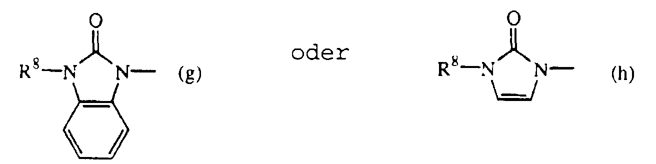 worin R⁸ Wasserstoff oder C₁₋₆-Alkyl bedeutet; steht.

3. Verbindung nach Anspruch 1, in der R¹ für Chlor steht.

4. Verbindung nach Anspruch 1, in der R² für Wasserstoff oder Methoxy steht.

5. Verbindung nach Anspruch 1, ausgewählt unter 4-Amino-5-chlor-2,3-dihydro-7-benzofurancarbonsäure-1-[(tetrahydro-2-furanyl)methyl]-4-piperidinylester; 5-Amino-6-chlor-3,4-dihydro-2,2-dimethyl-2*H*-1-benzopyran-8-carbonsäure-1-[(tetrahydro-2-furanyl)methyl]-4-piperidinylester; 4-Amino-5-chlor-2,3-dihydro-7-benzofurancarbonsäure-1-(3-methoxypropyl)-4-piperidinylester; 4-Amino-5-chlor-2,3-dihydro-7-benzofurancarbonsäure-1-[3-(2-methyl-1,3-dioxolan-2-yl)propyl]-4-piperidinylester; 4-Amino-5-chlor-2,3-dihydro-7-benzofurancarbonsäure-1-[3-(1-methylethoxy)propyl]-4-piperidinylester; 4-Amino-5-chlor-2,3-dihydro-7-benzofurancarbonsäure-1-[2-(2-hydroxyethoxy)ethyl]-4-piperidinylester; 4-Amino-5-chlor-2,3-dihydro-7-benzofurancarbonsäure-1-[3-(3-chlor-6-oxo-1(6*H*)pyridazinyl)propyl]-4-piperidinylester; 4-Amino-5-chlor-2,3-dihydro-7-benzofurancarbonsäure-1-(4-oxopentyl)-4-piperidinylester; 4-[[(4-Amino-5-chlor-2,3-dihydro-7-benzofuranyl)carbonyl]oxy]-1-piperidinbutansäureethylester und 4-Amino-5-chlor-2,3-dihydro-7-benzofurancarbonsäure-1-[2-(tetrahydro-2-furanyl)ethyl]-4-piperidinylester; einer stereochemisch isomeren Form davon oder einem pharmazeutisch unbedenklichen Säureadditionssalz davon.

6. Pharmazeutische Zusammensetzung, enthaltend eine therapeutisch wirksame Menge einer Verbindung nach Anspruch 1 und einen pharmazeutisch unbedenklichen Träger.

7. Verfahren zur Herstellung einer pharmazeutischen Zusammensetzung nach Anspruch 6, bei dem man die Verbindung nach Anspruch 1 innig mit einem pharmazeutisch unbedenklichen Träger vermischt.

8. Verbindung nach Anspruch 1 zur Verwendung als Arzneimittel.

9. Verwendung einer Verbindung nach Anspruch 1 zur Herstellung eines Medikaments für die Behandlung von Darmstörungen, die mit einer verringerten Beweglichkeit des Dickdarms einhergehen.

10. Verfahren zur Herstellung einer Verbindung nach Anspruch 1, bei dem man
a) ein Piperidin der Formel (II) mit einem Zwischenprodukt der Formel (III) N-alkyliert: 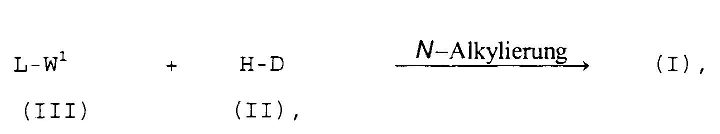 wobei L die in Anspruch 1 angegebenen Bedeutungen hat, W¹ eine geeignete Abgangsgruppe bedeutet und D für einen Rest der Formel 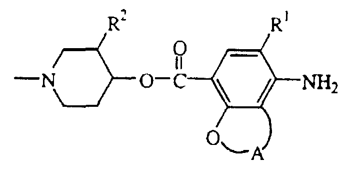 steht, worin R¹, R² und A die in Anspruch 1 angegebenen Bedeutungen haben;
b) einen Alkohol der Formel (IV) mit einer Carbonsäure der Formel (V) oder einem funktionellen Derivat davon, wie einem Acylhalogenid, einem symmetrischen oder gemischten Anhydrid oder einem Ester, umsetzt: 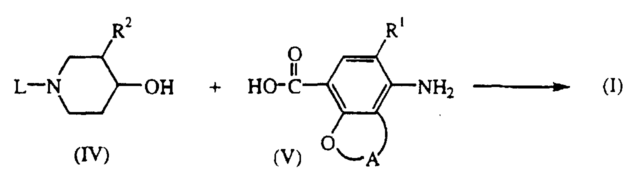 worin R¹, R², L und A die in Anspruch 1 angegebenen Bedeutungen haben;
und gegebenenfalls zum Umwandeln der Verbindungen der Formel (I) durch eine Reaktion zur Transformation funktioneller Gruppen ineinander und gegebenenfalls zum Umwandeln einer Verbindung der Formel (I) in ein therapeutisch wirksames nichttoxisches Säureadditionssalz oder umgekehrt zum Umwandeln eines Säureadditionssalzes mit Alkali in die Form der freien Base und/oder zur Herstellung stereochemisch isomerer Formen davon.

## Revendications

1. Composé de formule 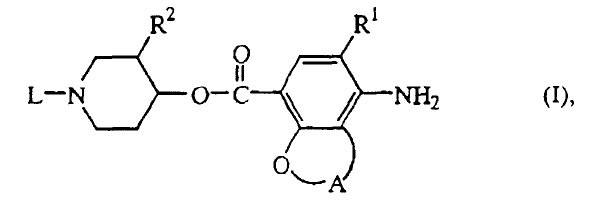 forme N-oxyde, sel d'addition pharmaceutiquement acceptable à un acide ou forme stéréochimiquement isomère de celui-ci, dans laquelle
R¹ est un halogéno ou un C₁₋₆-alkylsulfonylamino;
A représente un radical bivalent de formule :
-CH₂-CH₂- (a),
-CH₂-CH₂-CH₂- (b),
-CH=CH- (c),
dans les radicaux (a), (b) et (c), un ou deux atomes d'hydrogène peuvent être remplacés par un alkyle en C₁₋₆ ;
R² est un hydrogène ou un alkyloxy en C₁₋₆ ;
L est un radical de formule :
-Alk-R⁴ (d),
-Alk-O-R⁵ (e),
-Alk-NR⁶R⁷ (f),
Alk est un C₁₋₁₂-alcanediyle ;
R⁴ est un hydrogène ; un cyano ; un C₁₋₆-alkylcarbonyle ; un C₁₋₆-alkyloxycarbonyle ; un cycloalkyle en C₃₋₇ ; un C₁₋₆-alkylsulfinyle ; un C₁₋₆-alkylsulfonyle ; un phényle ou un phényle substitué par un halogéno ; un alkyle en C₁₋₆ ; ou un alkyloxy en C₁₋₆ ; un tétrahydrofurane ; un dioxolane ; un dioxolane substitué par un alkyle en C₁₋₆ ; un dioxane ; un dioxane substitué par un alkyle en C₁₋₆ ; une pyridine ; une pyridine substituée par un halogéno ou un alkyle en C₁₋₆ ; une pyridazine ; une pyridazine substituée par un ou deux substituants choisis parmi un halogéno, un alkyle en C₁₋₆, un hydroxy ; ou un radical de formule : 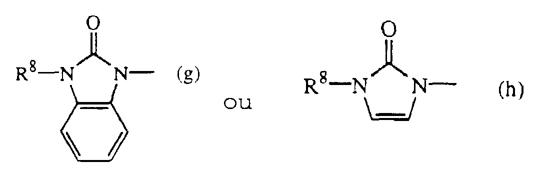 dans laquelle R⁸ est un hydrogène ou un alkyle en C₁₋₆ ;
R⁵ est un hydrogène ; un alkyle en C₁₋₆ ; un hydroxy-C₁₋₆-alkyle ; un C₁₋₆-alkylcarbonyle ; un phényle ou un phényle substitué par jusqu'à trois substituants choisis parmi un halogéno, un alkyle en C₁₋₆, un alkyloxy en C₁₋₆ ;
R⁶ est un hydrogène ou un alkyle en C₁₋₆ ;
R⁷ est un hydrogène ; un alkyle en C₁₋₆ ; un C₁₋₆-alkylcarbonyle ; un C₁₋₆-alkyloxycarbonyle ; une pyridazine ; une pyridazine substituée par un ou deux substituants choisis parmi un halogéno, un alkyle en C₁₋₆ , un hydroxy ; une pyrazine ; une pyrazine substituée par un ou deux substituants choisis parmi un halogéno, un alkyle en C₁₋₆, un hydroxy ;
à condition que lorsque A est un radical bivalent de formule (c) et L est un radical de formule (d), alors R⁴ n'est pas un hydrogène, un cyano, un C₁₋₆-alkylcarbonyle, un phényle ou un phényle substitué par un halogéno, un alkyle en C₁₋₆ ou un alkyloxy en C₁₋₆ ; ou lorsque A est un radical bivalent de formule (c) et L est un radical de formule (e), alors R⁵ n'est pas un hydrogène, un alkyle en C₁₋₆ ou un phényle ; ou lorsque A est un radical bivalent de formule (c) et L est un radical de formule (f), alors R⁷ n'est pas un C₁₋₆-alkylcarbonyle.

2. Composé selon la revendication 1, dans lequel R⁴ est un hydrogène, un cyano ; un C₁₋₆-alkylcarbonyle ; un C₁₋₆-alkylsulfinyle ; un C₁₋₆-alkylsulfonyle ; un phényle ou un phényle substitué par un halogéno ; un alkyle en C₁₋₆ ; ou un alkyloxy en C₁₋₆ ; un tétrahydrofurane ; un dioxolane ; un dioxolane substitué par un alkyle en C₁₋₆ ; un dioxane ; un dioxane substitué par un alkyle en C₁₋₆ ; une pyridine ; une pyridine substituée par un halogéno ou un alkyle en C₁₋₆ ; une pyridazine ; une pyridazine substituée par un ou deux substituants choisis parmi un halogéno, un alkyle en C₁₋₆, un hydroxy ; ou un radical de formule : 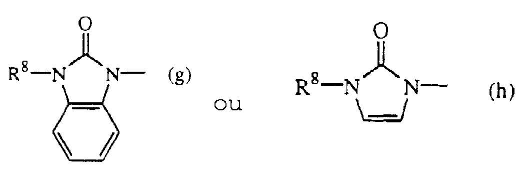 dans laquelle R⁸ est un hydrogène ou un alkyle en C_{1-6·}

3. Composé selon la revendication 1, dans lequel R¹ est un chloro.

4. Composé selon la revendication 1, dans lequel R² est un hydrogène ou un méthoxy.

5. Composé selon la revendication 1, le composé étant choisi parmi le 4-amino-5-chloro-2,3-dihydro-7-benzofuranécarboxylate de 1-[(tétrahydro-2-furanyl)méthyl]-4-pipéridinyle ; le 5-amino-6-chloro-3,4-dihydro-2,2-diméthyl-2H-1-benzopyrane-8-carboxylate de 1-[(tétrahydro-2-furanyl)méthyl]-4-pipéridinyle ; le 4-amino-5-chloro-2,3-dihydro-7-benzofuranecarboxylate de 1-(3-méthoxypropyl)-4-pipéridinyle ; le 4-amino-5-chloro-2,3-dihydro-7-benzofuranecarboxylate de 1-[3-(2-méthyl-1,3-dioxolan-2-yl)propyl)-4-pipéridinyle ; le 4-amino-5-chloro-2,3-dihydro-7-benzofuranecarboxylate de 1-[3-(1-méthyléthoxy)propyl]-4-pipéridinyle ; le 4-amino-5-chloro-2,3-dihydro-7-benzofuranecarboxylate de 1-[2-(2-hydroxyéthoxy)éthyl]-4-pipéridinyle ; le 4-amino-5-chloro-2,3-dihydro-7-benzofuranecarboxylate de 1-[3-(3-chloro-6-oxo-1(6*H*)-pyridazinyl)propyl]-4-pipéridinyle ; le 4-amino-5-chloro-2,3-dihydro-7-benzofuranecarboxylate de 1-(4-oxopentyl)-4-pipéridinyle ; le [[(4-amino-5-chloro-2,3-dihydro-7-benzofuranyl)carbonyl]oxy]-1-pipéridinebutanoate d'éthyle ; et le 4-amino-5-chloro-2,3-dihydro-7-benzofuranecarboxylate de 1-[2-(tétrahydro-2-furanyl)éthyl]-4-pipéridinyle ; une forme stéréochimiquement isomère de celui-ci ou un sel d'addition pharmaceutiquement acceptable de celui-ci à un acide.

6. Composition pharmaceutique comprenant une quantité thérapeutiquement efficace d'un composé selon la revendication 1 et d'un support pharmaceutiquement acceptable.

7. Procédé de préparation d'une composition pharmaceutique selon la revendication 6, dans lequel le composé selon la revendication 1 est mélangé intimement avec un support pharmaceutiquement acceptable.

8. Composé selon la revendication 1 destiné à être utilisé en tant que médicament.

9. Utilisation d'un composé selon la revendication 1 pour l'élaboration d'un médicament destiné au traitement de troubles intestinaux impliquant une motilité réduite du colon.

10. Procédé de préparation d'un composé selon la revendication 1, dans lequel
a) une pipéridine de formule (II) est N-alkylée avec un intermédiaire de formule (III), 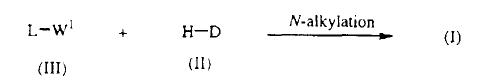 où L est tel que défini à la revendication 1, W¹ est un groupe partant approprié et D est un radical de formule 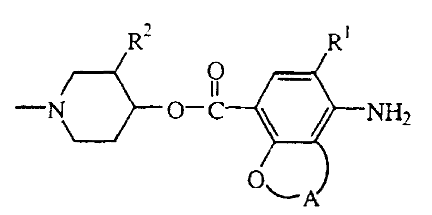 dans laquelle R¹, R² et A sont tels que définis à la revendication 1 ;
b) un alcool de formule (IV) est mis à réagir avec un acide carboxylique de formule (V) ou un dérivé fonctionnel de celui-ci, tel qu'un halogénure d'acyle, un anhydride symétrique ou mixte ou un ester ; 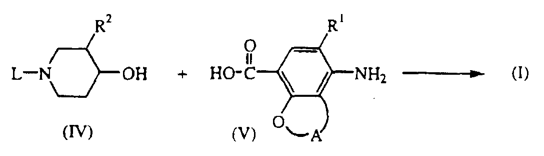 où R¹, R², L et A sont tels que définis à la revendication 1 ;
et éventuellement la transformation des composés de formule (I) les uns en les autres par une réaction de transformation de groupe fonctionnel ; et, si on le souhaite, la transformation d'un composé de formule (I) en un sel d'addition thérapeutiquement actif et non toxique, ou inversement, la transformation d'un sel d'addition à un acide en une forme de base libre avec un alcali ; et/ou la préparation de formes stéréochimiquement isomères de ceux-ci.
